# EUROPEAN PATENT APPLICATION

(11) **EP 4 843 597 A2**
(43) Date of publication of application: **25.11.2026**
(21) Application number: 26180673.1
(22) Date of filing: 22.05.2026
(51) Int. Cl.: A61M 16/04, A61B 1/267

(54) **ORAL AND NASAL TRACHEAL INTUBATION ASSISTANCE DEVICE**

(30) Priority: 01.04.2025 US 202519096793; 10.03.2026 US 202619561661
(71) Applicant: Stramiello, Joshua Anthony, Lakenheath, Brandon, Suffolk IP27 9PS (GB)
(72) Inventor: Stramiello, Joshua Anthony, Lakenheath, Brandon, Suffolk IP27 9PS (GB)
(74) Representative: Bewley, Ewan Stuart

(57) **Abstract**

An oral or nasal tracheal intubation assistance device for assisting in endotracheal intubation includes an esophageal plug portion and a ramped guidance portion. The oral or nasal tracheal intubation assistance device temporarily occludes the esophageal inlet while providing a guided pathway for endotracheal tube placement into the trachea. The oral or nasal tracheal intubation assistance device includes a handle with an ergonomic grip oriented perpendicular to the body via a curved transition portion. Two primary embodiments are disclosed: an oral version for orotracheal intubation and a nasal version for nasotracheal intubation. The ramped guidance portion includes a capture segment and a guidance segment that work in concert to redirect and guide the endotracheal tube toward the tracheal inlet. The oral tracheal intubation assistance device provides a standardized approach to both oral and nasal intubation procedures while improving first-pass success rates.

## Description

### Field

The present invention relates generally to medical devices for assisting in endotracheal intubation procedures. More specifically, the invention relates to devices for guiding the placement of endotracheal tubes during oral or nasal intubation procedures.

### Background

Endotracheal intubation is a critical medical procedure performed millions of times annually in operating rooms, emergency departments, and intensive care units worldwide. The procedure involves placing a breathing tube through either the mouth - orotracheal intubation - or nose - nasotracheal intubation - and into the trachea to establish a secure airway for mechanical ventilation. Success of this procedure is essential for patient survival in emergency situations and for airway management during surgical procedures.

Despite technological advances in video laryngoscopy, traditional direct laryngoscopy remains the primary method for endotracheal intubation. But this approach presents several challenges. For example, the practitioner must navigate complex throat anatomy, including the potential for the tube to enter the esophagus instead of the trachea. Failed first attempts at intubation can lead to tissue trauma, oxygen desaturation, and in severe cases, patient mortality.

The need exists for an oral or nasal tracheal intubation assistance device that can provide consistent, reliable guidance of endotracheal tubes into the trachea while preventing inadvertent esophageal intubation and injury to the cricoid cartilage, which is part of the anterior tracheal wall.

### Summary

The present invention addresses these needs by providing an oral or nasal tracheal intubation assistance device that temporarily occludes the esophageal inlet while simultaneously guiding an endotracheal tube into the trachea via a more anatomically correct pathway. The oral or nasal tracheal intubation assistance device includes an esophageal plug portion that sits at the upper esophageal sphincter and a ramped guidance portion that terminates at the supraglottis.

The invention provides an orotracheal intubation assistance device -O-TAD- or nasotracheal intubation -N-TAD- configured for insertion of an endotracheal tube through a patient's mouth.

The orotracheal intubation assistance device includes a handle with an elongated channel formed into an upper surface of the handle. The channel extends along a longitudinal axis of the handle from a proximal end of the handle toward a distal end of the handle. The channel defines a concave, trough-shaped profile in cross-section, the concave profile sized to receive and cradle a lower portion of the endotracheal tube. The channel is open along its entire length - the channel does not enclose or surround the endotracheal tube at any point. During use, the endotracheal tube rests within the channel, and is then free to be lifted away from the channel in a direction perpendicular to the longitudinal axis of the handle.

The channel of the handle forms the capture segment of the multipart ramp. The channel transitions from the handle to the body at the distal end of the handle, where the guidance segment continues the concave profile and curves away from the body to direct the endotracheal tube toward the trachea. The transition between the capture segment and the guidance segment is continuous, providing an uninterrupted guided pathway from the handle to the plug.

The multipart ramp extends across the full length of the tracheal intubation assistance device, from the proximal tip of the handle to the plug at the distal end of the body. Because the capture segment begins at the proximal tip of the handle, the practitioner loads the endotracheal tube onto the device at a position exterior to the patient's oral cavity. The endotracheal tube is then advanced along the multipart ramp in a single continuous motion, guided from the exterior loading position, through the oral cavity, and into the trachea. The handle serves a dual function: a lower surface provides a grip for the practitioner, and the upper surface provides the capture segment of the multipart ramp. This arrangement consolidates tube guidance and device manipulation into a single component.

The ramped guidance portion includes a capture segment that initially engages the endotracheal tube, followed by a guidance segment that alters the direction of the endotracheal tube to pass into the trachea. Unlike conventional tube guides that fully encircle the endotracheal tube, the ramps include an open side extending along their entire length. This open-sided design allows the oral tracheal intubation assistance device to only partially surround the endotracheal tube during guided insertion, enabling lateral separation of the device from the tube after successful intubation. Specifically, the practitioner can readily withdraw the oral tracheal intubation assistance device by moving it sideways away from the secured endotracheal tube, eliminating the need to slide the device back over the full length of the tube, which improves the safety of airway securement.

Restated, the multipart ramp does not fully surround the endotracheal tube at any position - the device can be removed from the patient at any time without dislodging or passing over the endotracheal tube.

The channel contacts less than half of the circumference of the endotracheal tube at any point along the length of the channel. In one embodiment, the channel contacts between approximately one-quarter and one-third of the circumference of the endotracheal tube. The first edge and the second edge of the multipart ramp define lateral boundaries of the channel. The first edge and the second edge are spaced apart to define the open side of the multipart ramp. The open side permits the practitioner to withdraw the tracheal intubation assistance device laterally, in a direction transverse to the longitudinal axis of the endotracheal tube, without displacing the endotracheal tube from its seated position within the trachea.

The nasal version of the tracheal intubation assistance device includes an ergonomically designed handle with a grip portion oriented perpendicular to a longitudinal axis of the body, the direction change accomplished via a curved transition portion. The handle attachment point is offset from the central axis of the ramp to prevent interference with endotracheal tube placement.

In the oral version, the handle is integrated with the proximal end of the body, and the plug is integrated with the distal end of the body.

In a resting configuration, the handle and body are aligned along a common longitudinal axis, and the body lower face is flat. During insertion, the flexible handle deflects into a flexed configuration, curving the body lower face and producing a compound curve along the multipart ramp that follows the patient's oral-pharyngeal anatomy. The concave cross-sectional profile and the open side of the multipart ramp are maintained throughout the range of flexion.

For oral intubation, the device requires visualization via direct or indirect laryngoscopy. The oral embodiment is not intended for use in blind intubation.

Multiple size variations accommodate different patient anatomies, including pediatric models. The oral tracheal intubation assistance device is constructed of flexible, biocompatible materials and has demonstrated efficacy in both anatomical models and cadaver studies.

The flexible material is preferably a polymer with a Shore hardness of approximately 80A. This hardness provides flexibility for anatomical conformity while maintaining structural integrity for endotracheal tube guidance. Anticipated methods of manufacturing include 3D printing and injection molding.

### Brief Description of the Drawings

The invention can be best understood by those having ordinary skill in the art by reference to the following detailed description when considered in conjunction with the accompanying drawings in which:
Figure 1 illustrates a first isometric view of the nasal embodiment of the intubation assistance device.
Figure 2 illustrates a first side view of the nasal embodiment of the intubation assistance device.
Figure 3 illustrates a second isometric view of the nasal embodiment of the intubation assistance device.
Figure 4 illustrates a front view of the nasal embodiment of the intubation assistance device.
Figure 5 illustrates a third isometric view of the nasal embodiment of the intubation assistance device.
Figure 6 illustrates a cross-sectional view of the nasal embodiment in use of the intubation assistance device.
Figure 7 illustrates a second side view of the nasal embodiment of the intubation assistance device.
Figure 8 illustrates a rear view of the nasal embodiment of the intubation assistance device.
Figure 9 illustrates a second front view of the nasal embodiment of the intubation assistance device.
Figure 10 illustrates a top view of the nasal embodiment of the intubation assistance device.
Figure 11 illustrates a bottom view of the nasal embodiment of the intubation assistance device.
Figure 12 illustrates a fourth isometric view of the nasal embodiment of the intubation assistance device.
Figure 13 illustrates a first isometric view of the oral embodiment of the intubation assistance device.
Figure 14 illustrates a side view of the oral embodiment of the intubation assistance device.
Figure 15 illustrates a top view of the oral embodiment of the intubation assistance device.
Figure 16 illustrates a bottom view of the oral embodiment of the intubation assistance device.
Figure 17 illustrates an isometric view of the oral embodiment of the intubation assistance device.
Figure 18 illustrates a rear view of the oral embodiment of the intubation assistance device.
Figure 19 illustrates a front view of the oral embodiment of the intubation assistance device.
Figure 20 illustrates a cross-sectional view of the oral embodiment in use of the intubation assistance device.

### Detailed Description

Reference will now be made in detail to the presently preferred embodiments of the invention, examples of which are illustrated in the accompanying drawings. Throughout the following detailed description, the same reference numerals refer to the same elements in all figures.

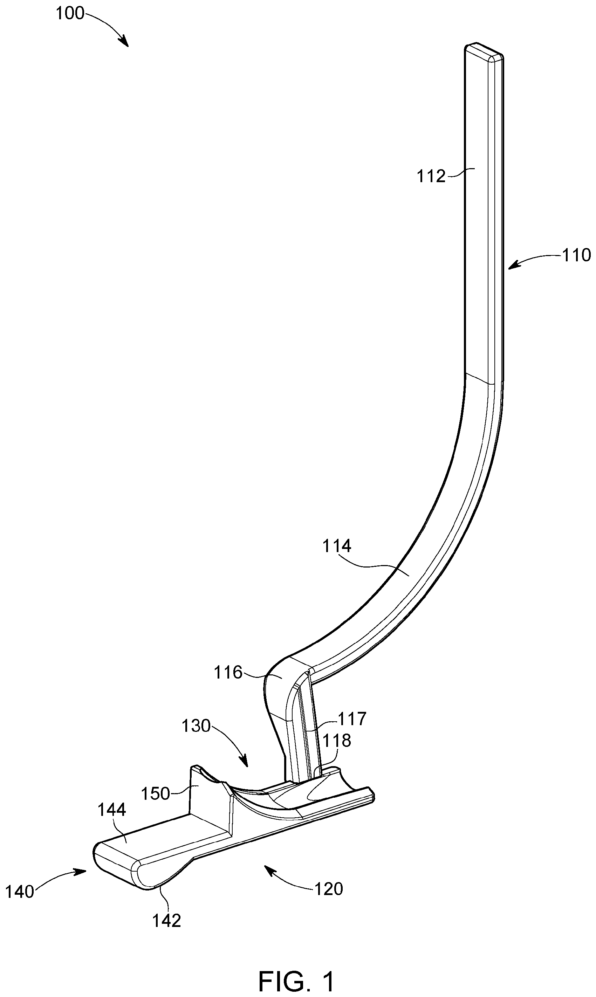

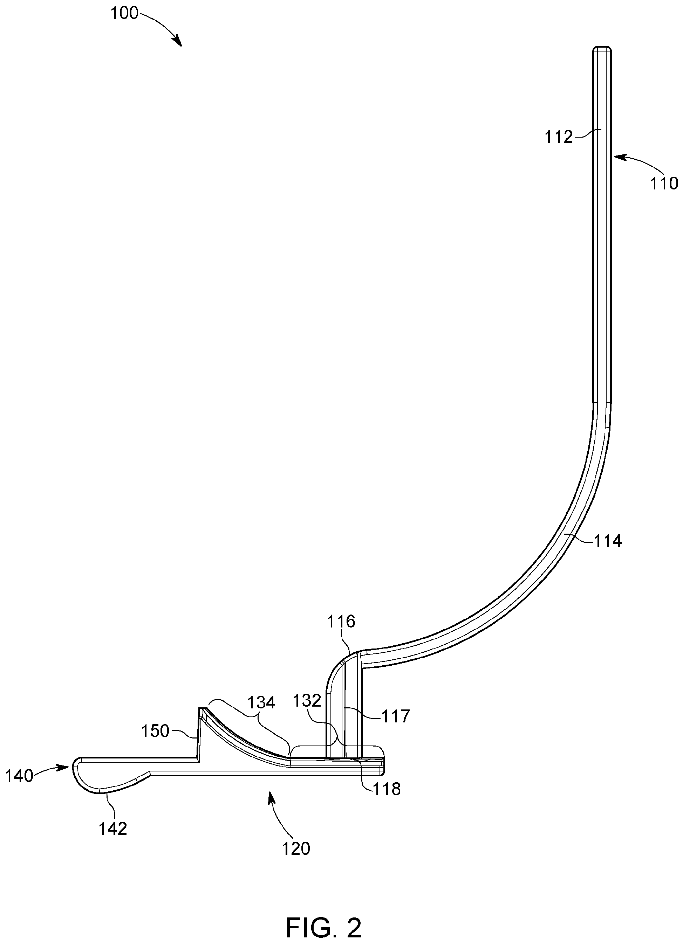

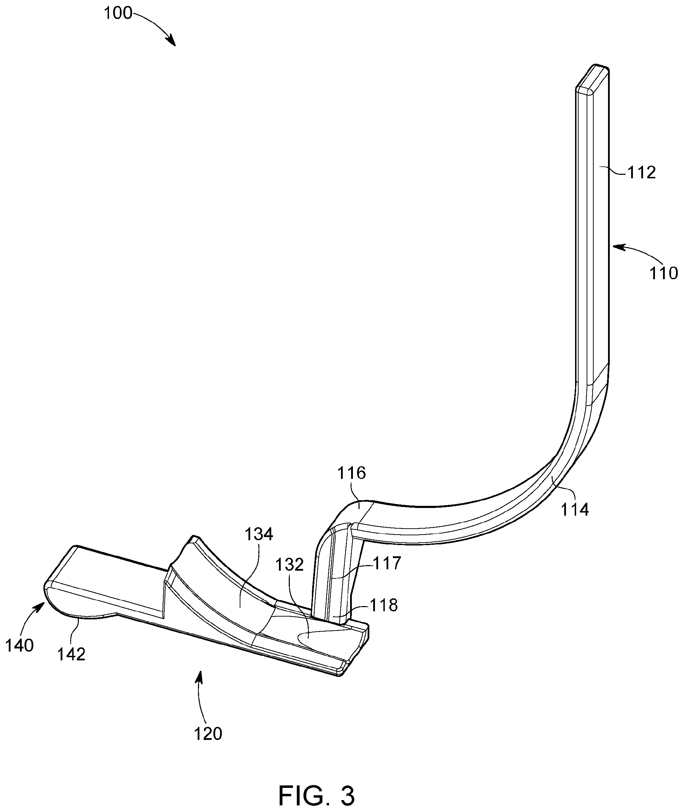

Referring to Figures 1, 2, and 3, a first isometric view, a first side view, and a second isometric view of the nasal embodiment of the intubation assistance device are shown.

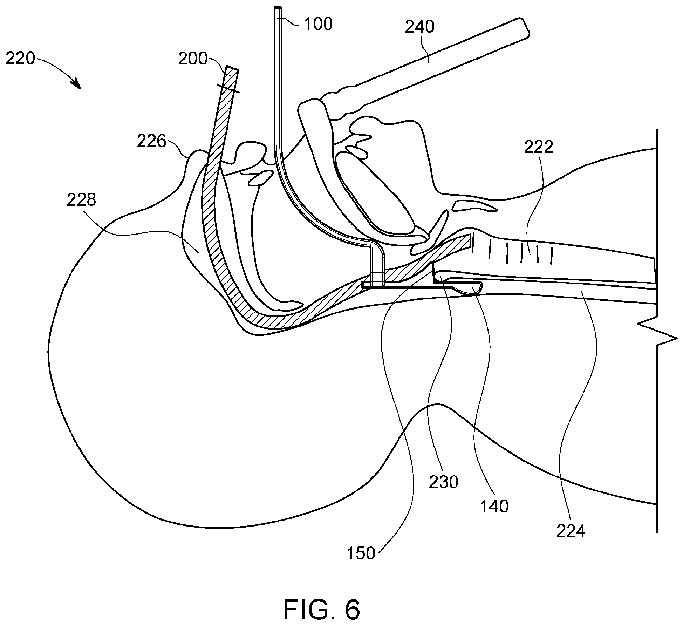

The tracheal intubation assistance device 100 is shown with an offset handle 110. This first embodiment of the tracheal intubation assistance device 100 is intended for use with insertion of the endotracheal tube 200 (see Figure 6) through the nose. The offset handle 110 prevents interference between any portion of the offset handle 110 and the endotracheal tube 200 during insertion of the endotracheal tube 200.

The offset handle 110 includes a grip 112 for the practitioner's hand, connected to a curved transition 114 that gradually accomplishes an approximate 90° shift in angle toward a plane parallel to the body 120. The handle continues via a connection to an angular transition 116 accomplishing an additional 90° shift in angle to the vertical transition 117 and finally to the offset attachment point 118.

The body 120 of the tracheal intubation assistance device 100 includes the multipart ramp 130 with capture segment 132, where the endotracheal tube 200 (see Figure 6) first interacts with the multipart ramp 130, and the guidance segment 134, which alters the direction of the endotracheal tube 200 to pass into the trachea.

An end of the body includes the plug 140 that blocks passage of the endotracheal tube 200 into the esophagus. The plug 140 includes the plug protrusion 142, which acts to increase the height of the plug in the Z-direction to better fit the esophagus, without requiring a consistent increase in thickness of the entire body 120, which would make manipulation of the tracheal intubation assistance device 100 more difficult.

The plug upper face 144 is preferably flat, allowing for passage of the plug 140 into the hypopharynx at the junction of the supraglottis and hypopharynx, the insertion stop 150 resting against the arytenoids to prevent or limit insertion beyond the desired depth. The plug obstructs the esophageal inlet, the multipart ramp 130 guiding insertion of a breathing tube to the glottis and into the trachea.

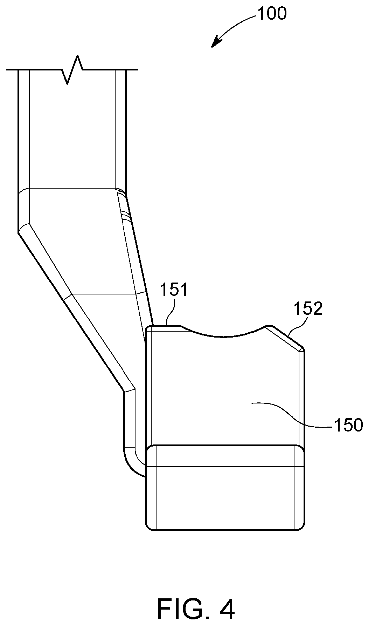

Referring to Figure 4, a front view of the nasal embodiment of the intubation assistance device is shown.

The tracheal intubation assistance device 100 is shown with the insertion stop 150 including a clipped corner 152. As compared to the non-clipped corner 151, the clipped corner 152 of the insertion stop 150 eases insertion and removal of the tracheal intubation assistance device 100 where corners of the insertion stop 150 would catch on the patient's anatomy.

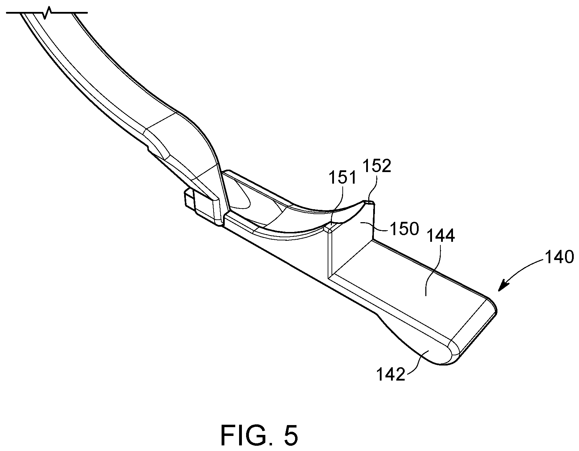

Referring to Figure 5, a third isometric view of the nasal embodiment of the intubation assistance device is shown.

The plug 140 is shown with plug protrusion 142 and plug upper face 144.

The insertion stop 150 is shown with non-clipped corner 151 and clipped corner 152.

Referring to Figure 6, a cross-sectional view of the first embodiment in use of the oral tracheal intubation assistance device is shown.

The tracheal intubation assistance device 100 is shown being used with a patient 220. A laryngoscope 240 is used to open the airway in preparation for insertion of the endotracheal tube 200. The endotracheal tube 200 is passed through the nose 226, the nasal cavity 228, and then guided by the tracheal intubation assistance device 100. The insertion stop 150 rests against the arytenoid cartilages (supraglottis) 230, preventing over-insertion. The plug 140 rests within the hypopharynx 224, blocking the upper esophageal sphincter and ensuring that the endotracheal tube 200 is passed into the trachea 222.

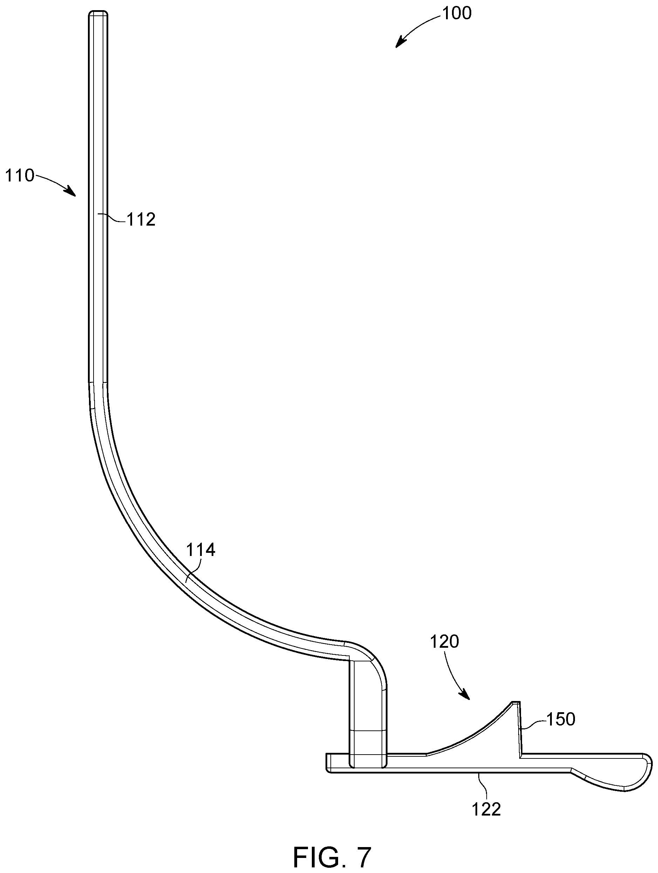

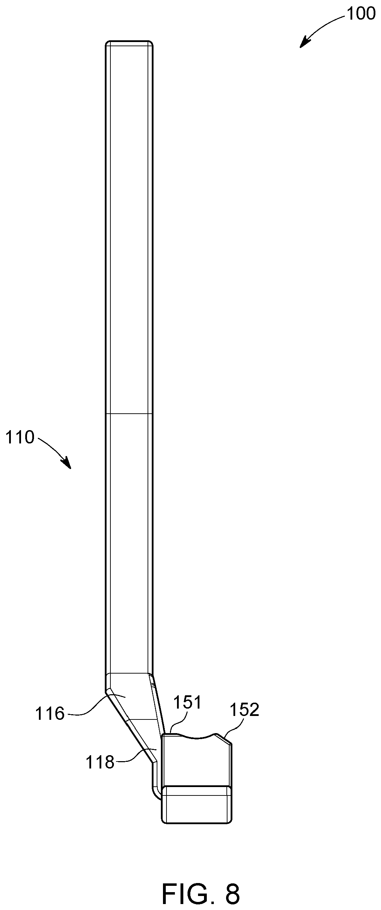

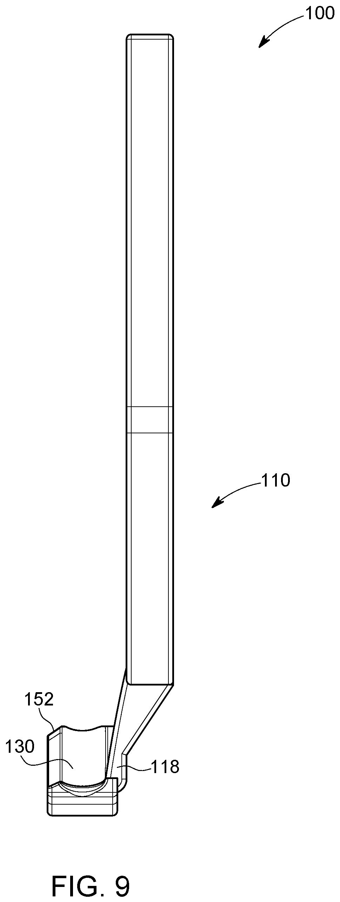

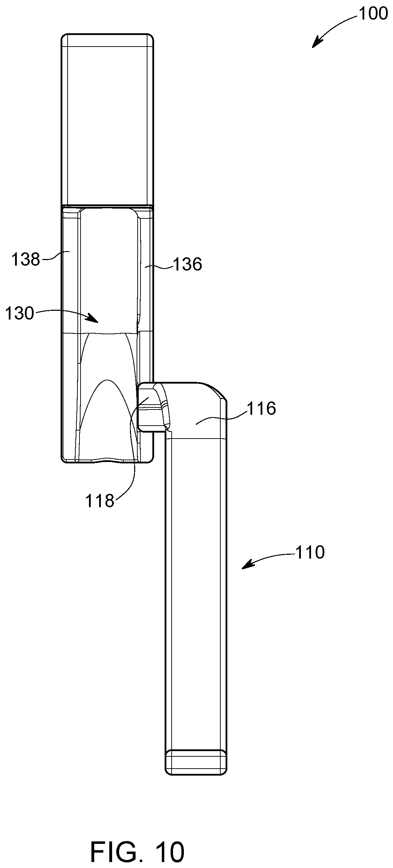

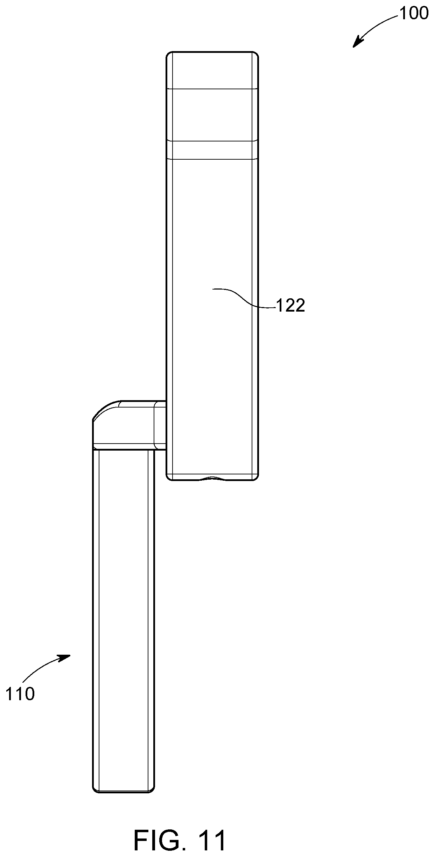

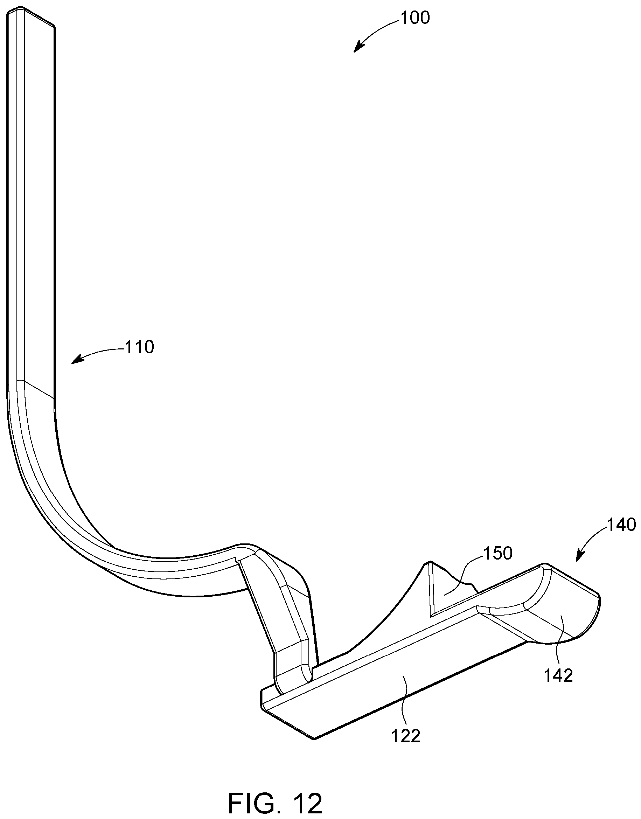

Referring to Figures 7 through 12, multiple views of the first embodiment of the oral tracheal intubation assistance device are shown.

The offset handle 110 of the tracheal intubation assistance device 100 is visible in multiple figures. The offset handle 110, including the offset attachment point 118 and angular transition 116, ensures that the grip 112 is laterally offset from the multipart ramp 130. By having the offset handle 110 fully laterally offset from the multipart ramp 130, the endotracheal tube 200 (see Figure 6) passes across the multipart ramp 130 without any interference with the offset handle 110 or its connection points.

The body includes body lower face 122, preferably shaped as a flat, planar surface. This surface is inset with respect to the plug 140, specifically the plug protrusion 142, easing insertion of the tracheal intubation assistance device 100 into the patient by providing additional clearance with respect to the patient's airway.

The insertion stop 150 with clipped corner 152 and non-clipped corner 151 is also visible. The clipped corner 152 reduces the interference points during insertion and removal of the tracheal intubation assistance device 100.

The multipart ramp 130 includes the curved, scooping, self-centering central section, assisting with guidance and automatic centering of the endotracheal tube 200 during insertion. Adjacent to the multipart ramp 130 are the first edge 136 and the second edge 138. Contact between the endotracheal tube 200 and either the first edge 136 or the second edge 138 helps to push the endotracheal tube 200 back toward the center of the multipart ramp 130, ensuring guidance during insertion.

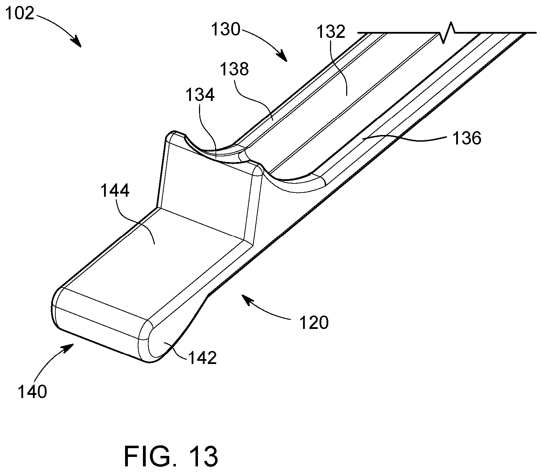

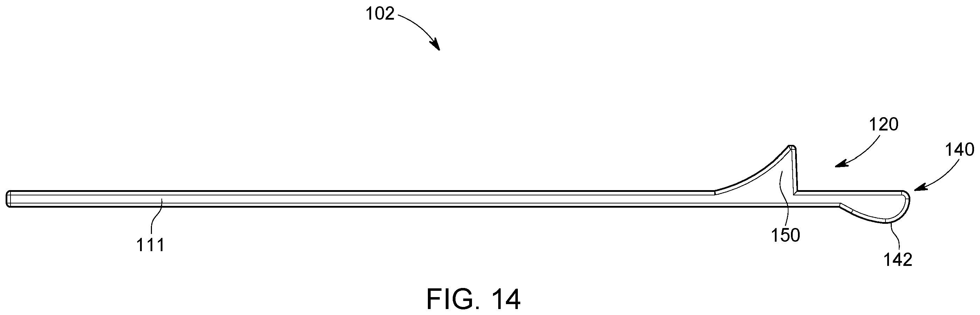

Referring to Figures 13 and 14, a first isometric view and a side view of the oral embodiment of the intubation assistance device are shown.

The oral embodiment of the intubation assistance device 102 is intended for use with oral intubation, rather than nasal intubation. The handle 111 is aligned with the body 120, and the multipart ramp 130 is integrated into the handle 111. The multipart ramp 130 again includes the capture segment 132 and guidance segment 134. By integrating the multipart ramp 130 with the handle 111, during use the endotracheal tube 200 passes along the multipart ramp 130 from the starting position outside of the patient, through the mouth, and ultimately into the trachea.

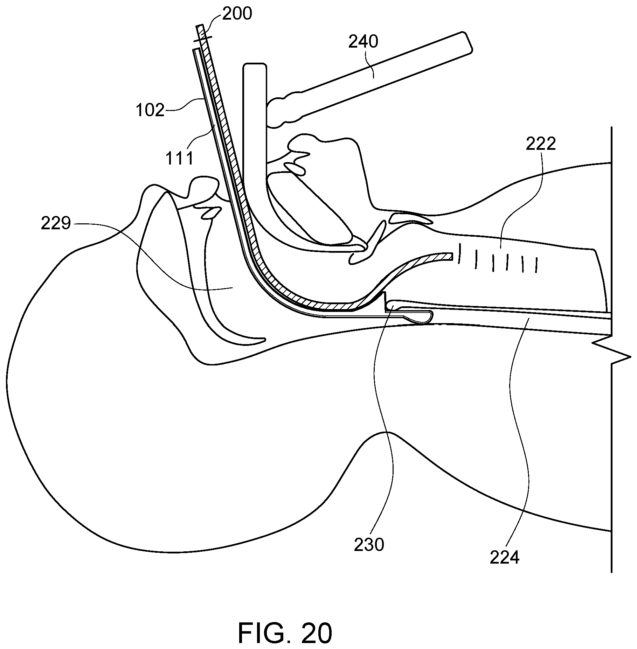

The multipart ramp 130 extends from the proximal end of the handle 111 to the distal end of the body 120. The capture segment 132 begins at the proximal tip of the handle 111 and is coextensive with the handle 111 along the full length of the handle 111. Because the capture segment 132 begins at the proximal tip of the handle 111, the practitioner places the endotracheal tube 200 (see Figure 20) into the multipart ramp 130 at a location that is exterior to the patient's oral cavity 229 (see Figure 20) during use. The endotracheal tube 200 is guided from this exterior starting position, through the oral cavity 229, and into the trachea 222 (see Figure 20) without leaving the multipart ramp 130 at any point along the insertion path. The multipart ramp 130 provides an uninterrupted guided pathway across the full insertion distance.

The handle 111 serves a dual function. A lower surface of the handle 111 provides a grip surface for the practitioner's hand. The upper surface of the handle 111 simultaneously provides the capture segment 132 of the multipart ramp 130. The practitioner holds the handle 111 from below and advances the endotracheal tube 200 along the upper surface of the handle 111 from above. This dual-function arrangement eliminates the need for a separate tube guide or stylet, consolidating the gripping function and the guidance function into a single structure.

To accomplish these functions, the handle 111 includes a channel formed into an upper surface of the handle 111. The channel is a multipart ramp 130, formed from the combination of the capture segment 132 and the guidance segment 134. The multipart ramp 130 extends along a longitudinal axis of the handle 111 from a proximal end of the handle 111 toward a distal end of the handle 111. The multipart ramp 130 has a concave cross-sectional profile defining a trough shape. The concave cross-sectional profile is configured to receive and cradle a lower portion of the endotracheal tube 200 (see Figure 20). The multipart ramp 130 does not enclose or surround the endotracheal tube 200. The endotracheal tube 200 rests within the multipart ramp 130 and contacts only its concave inner surface.

The multipart ramp 130 includes a first edge 136 and a second edge 138. The first edge 136 and the second edge 138 define the lateral boundaries of the multipart ramp 130. The first edge 136 and the second edge 138 are spaced apart from one another such that the multipart ramp 130 remains open between the first edge 136 and the second edge 138. The open configuration of the multipart ramp 130 permits the endotracheal tube 200 to be separated from the multipart ramp 130 in a lateral direction.

The capture segment 132 of the multipart ramp 130 is coincident with the handle 111. At the distal end of the handle 111, the capture segment 132 transitions to the guidance segment 134 of the multipart ramp 130. The transition between the capture segment 132 and the guidance segment 134 is continuous and smooth. The guidance segment 134 maintains the concave cross-sectional profile of the capture segment 132 while curving away from the body 120 to redirect the endotracheal tube 200 toward the trachea 222 (see Figure 20).

The handle 111 is formed from a flexible material, allowing it to bend during insertion of the oral tracheal intubation assistance device 102. The result is a gradual angular change in the multipart ramp 130, easing the process of guiding the endotracheal tube 200 (see Figure 20) into the patient's trachea.

In a resting configuration, the handle 111 and the body 120 are aligned along a common longitudinal axis. The body lower face 122 is flat and planar in the resting configuration. The multipart ramp 130 extends from the proximal tip of the handle 111 to the guidance segment 134 adjacent to the plug 140 in a substantially straight line along the common longitudinal axis, with the guidance segment 134 curving away from the body 120 at the distal end.

During insertion, the practitioner applies force to the handle 111. The flexible material of the handle 111 permits the handle 111 to deflect from the resting configuration into a flexed configuration. In the flexed configuration, the handle 111 is angled relative to the body 120, and the body lower face 122 curves. The angular relationship between the capture segment 132 and the guidance segment 134 changes from the resting configuration to the flexed configuration. The flexion produces a compound curve along the multipart ramp 130 that follows the oral-pharyngeal anatomy of the patient 220 (see Figure 20), easing advancement of the endotracheal tube 200 (see Figure 20) from the oral cavity 229 (see Figure 20) toward the trachea 222 (see Figure 20).

Throughout the range of flexion from the resting configuration to the flexed configuration, the concave cross-sectional profile of the multipart ramp 130 is maintained. The open side of the multipart ramp 130, defined by the first edge 136 and the second edge 138, is also maintained throughout the range of flexion. The endotracheal tube 200 remains seated within and guided by the multipart ramp 130 during flexion of the handle 111.

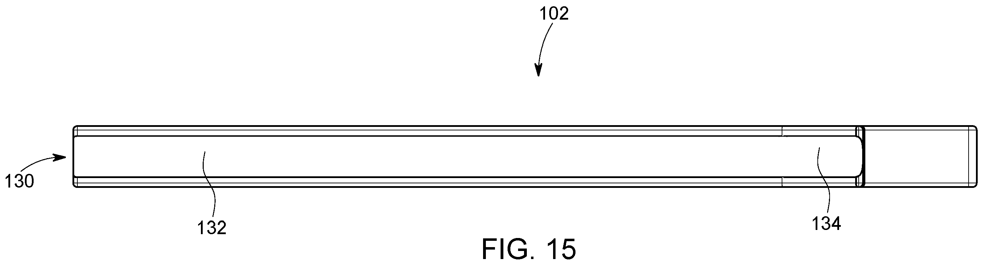

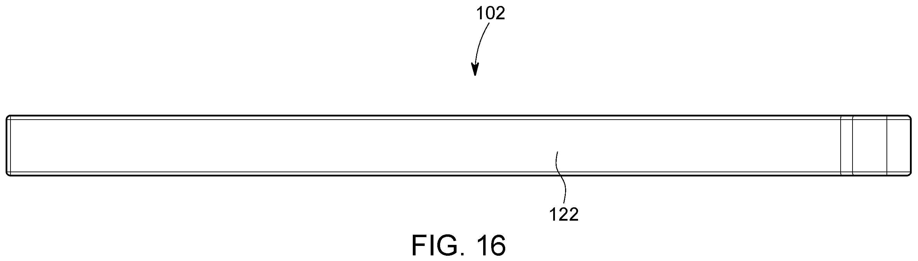

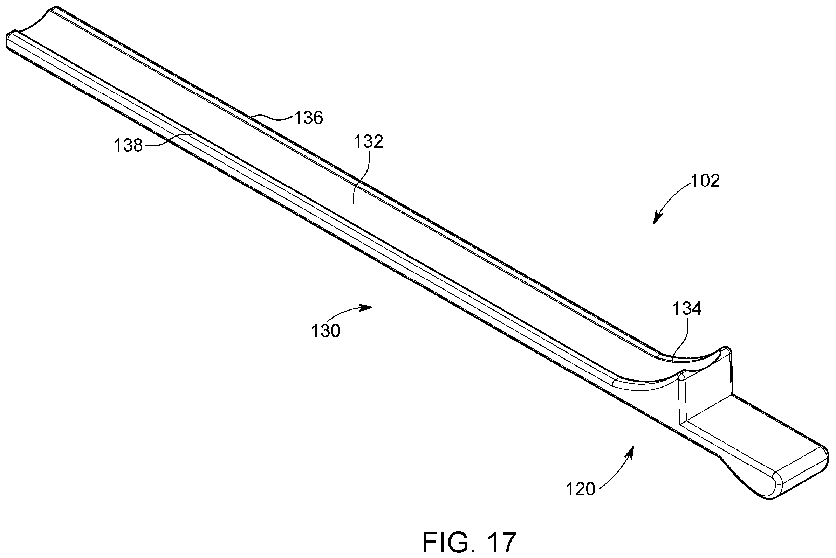

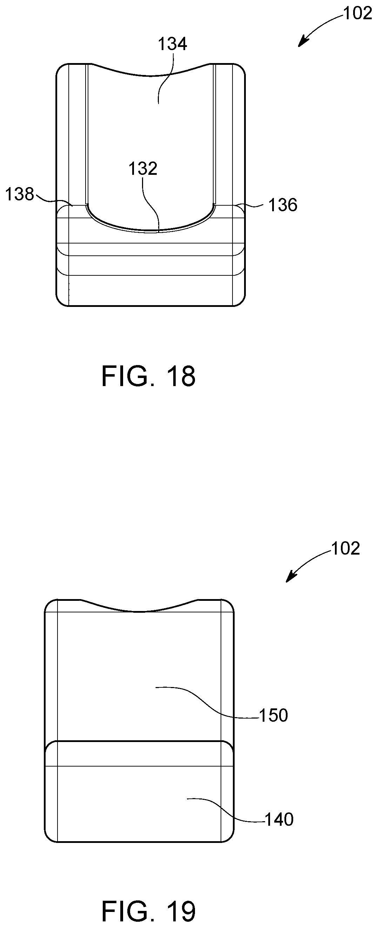

Referring to Figures 15 through 19, multiple views of the oral embodiment of the intubation assistance device are shown.

The oral embodiment of the intubation assistance device 102, intended for use in oral intubation, is shown with multipart ramp 130 including capture segment 132 and guidance segment 134. The handle 111 is combined with the capture segment 132 of the multipart ramp 130, controlling the path of the endotracheal tube 200.

The body 120 again includes body lower face 122 that is substantially planar in its resting, flat position. Again, the handle 111 is formed from a flexible material and therefore the body lower face will curve as pressure is applied to the handle 111.

The multipart ramp 130 includes a curved inner surface, defining the concave cross-sectional profile. The curved inner surface acts to center the endotracheal tube 200 during insertion. As the endotracheal tube 200 advances along the multipart ramp 130, contact between the endotracheal tube 200 and the curved inner surface guides the endotracheal tube 200 toward a centerline of the multipart ramp 130. The concave cross-sectional profile is substantially U-shaped, with a radius of curvature preferably selected to correspond to an outer diameter of the endotracheal tube 200. In one embodiment, the radius of curvature of the multipart ramp 130 is greater than a radius of the endotracheal tube 200, providing clearance for the endotracheal tube 200 to slide freely along the multipart ramp 130 while maintaining lateral centering.

The upper surface of the multipart ramp 130 preferably contacts less than half of a circumference of the endotracheal tube 200 at any cross-section along its length. The first edge 136 and the second edge 138 of the multipart ramp 130 terminate at a height that is below a midline of the endotracheal tube 200 when the endotracheal tube 200 is seated within the multipart ramp 130. This configuration ensures that the multipart ramp 130 cradles the endotracheal tube 200 from below without enclosing the endotracheal tube 200.

The multipart ramp 130 is open, meaning that the multipart ramp 130 does not enclose the endotracheal tube 200 that is being guided into the patient. As a result, the oral embodiment of the intubation assistance device 102 is readily removable from the patient.

The insertion stop 150 is again shown.

Referring to Figure 20, a cross-sectional view of the second embodiment in use of the oral tracheal intubation assistance device is shown.

The oral embodiment of the intubation assistance device 102 is shown being used with a patient 220. A laryngoscope 240 is used to open the airway in preparation for insertion of the endotracheal tube 200. The endotracheal tube 200 is passed through the oral cavity 229, guided by the oral embodiment of the intubation assistance device 102. The insertion stop 150 rests against the arytenoid cartilages (supraglottis) 230, preventing over-insertion. The plug 140 rests within the hypopharynx 224, ensuring that the endotracheal tube 200 is passed into the trachea 222.

The handle 111 of the oral embodiment of the intubation assistance device 102 curves to guide the endotracheal tube 200 as it moves toward the trachea 222.

The endotracheal tube 200 rests within the multipart ramp 130 of the handle 111 as the endotracheal tube 200 passes through the oral cavity 229. A proximal portion of the handle 111 remains exterior to the oral cavity 229 during use. The capture segment 132 at the proximal portion of the handle 111 provides an initial loading position for the endotracheal tube 200 that is outside the patient 220. The endotracheal tube 200 is placed into the capture segment 132 at this exterior position, aligned within the concave cross-sectional profile, and then advanced distally along the multipart ramp 130 in a single continuous motion. The endotracheal tube 200 remains in contact with the multipart ramp 130 from the exterior loading position, through the oral cavity 229, to the trachea 222. At no point during advancement does the endotracheal tube 200 leave the multipart ramp 130 or traverse an unguided gap.

The multipart ramp 130 maintains the endotracheal tube 200 in a centered position along the multipart ramp 130. As the handle 111 flexes, the capture segment 132 flexes with the handle 111, maintaining the concave cross-sectional profile throughout the range of flexion. The open configuration of the multipart ramp 130 permits the practitioner to withdraw the oral embodiment of the intubation assistance device 102 in a lateral direction after successful placement of the endotracheal tube 200, without displacing the endotracheal tube 200 from the trachea 222.

Equivalent elements can be substituted for the ones set forth above such that they perform in substantially the same manner in substantially the same way for achieving substantially the same result.

## Claims

1. A nasotracheal intubation assistance device for guiding insertion of an endotracheal tube into a patient's trachea, comprising:
a body with a proximal end and a distal end;
the body including a multipart ramp, the multipart ramp comprising a capture segment and a guidance segment, wherein the multipart ramp is configured to only partially surround the endotracheal tube;
the body including a body lower face with a flat surface;
the capture segment at the proximal end of the body where the endotracheal tube initially engages the device, the capture segment parallel to the body lower face;
the guidance segment curved away from the body and configured to direct the endotracheal tube toward the patient's trachea;
a plug connected to the body;
the plug at the distal end of the body;
the plug configured to position at an upper esophageal sphincter and obstruct outflow of stomach contents;
a fixed offset handle connected to the body at an offset attachment point, the offset attachment point between the proximal end of the body and the distal end of the body, the offset attachment point positioned laterally to one side of the multipart ramp to prevent interference of the multipart ramp with the endotracheal tube during removal of the multipart ramp after endotracheal tube placement;
wherein the multipart ramp further comprises a first edge and a second edge defining an open side extending along a full length of the multipart ramp, the open side allowing lateral removal of the nasotracheal intubation assistance device from a position in contact with the endotracheal tube to a position separated from the endotracheal tube.

2. The nasotracheal intubation assistance device of claim 1, wherein the plug further comprises a plug protrusion and a plug upper face;
the plug upper face having a flat, planar surface that is parallel to the body lower face;
the plug protrusion extending past the body lower face; and
the plug protrusion extending outward, away from the flat, planar surface of the plug upper face.

3. The nasotracheal intubation assistance device of claim 1, wherein the fixed offset handle comprises:
a grip;
a curved transition; and
a vertical transition connecting the curved transition to the offset attachment point.

4. The nasotracheal intubation assistance device of claim 3, wherein the grip extends substantially perpendicular to a longitudinal axis of the body.

5. The nasotracheal intubation assistance device of claim 2, further comprising:
an insertion stop configured to limit insertion depth by being configured to rest against arytenoids to prevent or limit insertion beyond a desired depth;
the insertion stop perpendicular to the plug upper face and perpendicular to the body lower face.

6. The nasotracheal intubation assistance device of claim 5, wherein the insertion stop comprises a non-clipped corner and a clipped corner.

7. A tracheal intubation assistance device for guiding insertion of an endotracheal tube into a patient's trachea, comprising:
a body comprising a body lower face, a proximal end and a distal end;
the body lower face having a planar surface;
a plug connected to the body at the distal end, the plug configured to rest at an upper esophageal sphincter;
the plug including a plug upper face, the plug upper face shaped to create a seal against the upper esophageal sphincter;
the plug extending past the body lower face;
a multipart ramp connected to the body, the multipart ramp comprising:
a capture segment at the proximal end configured to initially engage the endotracheal tube;
a guidance segment configured to alter a direction of the endotracheal tube to pass into the patient's trachea while contacting less than half of a circumference of the endotracheal tube; and
a first edge and a second edge defining an open side extending along a length of the multipart ramp, the open side enabling lateral removal of the device from the endotracheal tube; and
a handle;
the handle inline with the body;
the handle having a proximal end and a distal end;
the capture segment formed from an upper surface of the handle;
the capture segment starting at the proximal end of the handle and transitioning to the body at the distal end of the handle.

8. The tracheal intubation assistance device for guiding insertion of an endotracheal tube into a patient's trachea of claim 7, further comprising:
an insertion stop configured to limit insertion depth by being configured to rest against arytenoids to prevent or limit insertion beyond a desired depth;
the insertion stop perpendicular to the plug upper face.

9. The tracheal intubation assistance device for guiding insertion of an endotracheal tube into a patient's trachea of claim 7, wherein the device is sized for orotracheal intubation and configured for insertion through an oral cavity of a patient.

10. The tracheal intubation assistance device for guiding insertion of an endotracheal tube into a patient's trachea of claim 7, wherein the open side permits removal of the tracheal intubation assistance device in a direction transverse to a longitudinal axis of the endotracheal tube without displacing the endotracheal tube from the patient's trachea.
